# EUROPÄISCHE PATENTSCHRIFT

(11) **EP 1 171 203 B1**
(45) Veröffentlichungstag und Bekanntmachung des Hinweises auf die Patenterteilung: **02.06.2004**
(21) Anmeldenummer: 00909326.1
(22) Anmeldetag: 06.03.2000
(51) Int. Cl.: A61P 9/02, A61K 31/517

(54) **ARZNEIMITTEL, ENTHALTEND DOXAZOSIN-MESYLAT DER KRISTALLMODIFIKATION D**
MEDICAMENTS CONTAINING DOXAZOSIN-MESYLATE OF CRYSTALLINE MODIFICATION D
MEDICAMENTS CONTENANT DU MESYLATE DE DOXAZOSINE A MODIFICATION CRISTALLINE D

(30) Priorität: 19.03.1999 DE 19912573
(43) Veröffentlichungstag der Anmeldung: 16.01.2002
(73) Patentinhaber: Chemische Fabrik Berg GmbH, 06749 Bitterfeld (DE)
(72) Erfinder: THYES, Marco, D-67061 Ludwigshafen (DE); EINIG, Heinz, D-67433 Neustadt (DE); KLEIN, Peter, D-67134 Birkenheide (DE); HIX, Dieter, D-67229 Grosskarlbach (DE)
(74) Vertreter: Graf von Stosch, Andreas, Dr. rer. nat.
(86) Internationale Anmeldenummer: PCT/EP2000/001938
(87) Internationale Veröffentlichungsnummer: WO 2000/056293

(56) Entgegenhaltungen:
- EP-A- 0 848 001
- EP-A- 0 849 264
- EP-A- 0 849 265
- EP-A- 0 849 266
- WO-A-00/55157
- WO-A-99/35143
- DE-A- 19 912 063
- GRCMAN, M. ET AL: "Study of polymorphism of 1-(4-amino-6,7-dimethoxy-2-quinazolinyl)-4 - [(2,3-dihydro-1,4-benzodioxin-2-yl)carbony l]-piperazine monomethanesulfonate" FARM. VESTN. (LJUBLJANA) (1997), 48(POS. STEV.), 292-293 , XP000965554
- THOMBRE, A. G. ET AL: "In vitro/in vivo correlations of sustained-release coated multiparticulate formulations of doxazosin." INT. J. PHARM. (1994), 111(2), 181-9 , XP000964741

## Beschreibung

Die vorliegende Erfindung betrifft ein Arzneimittel, enthaltend Doxazosin-Mesylat in der Kristallmodifikation D.

Doxazosin (= 4-Amino-2-[4-(1,4-benzodioxan-2-carbonyl)piperazin-1-yl]-6,7-dimethoxychinazolin) ist eine bekannte Substanz (Merck-Index 12. Auflage 1996, Nr. 3489), die den Blutdruck senkt. Die Substanz wird überwiegend in der Form des Monomesylats verwendet, welches in kristalliner Form in mehreren Modifikationen auftritt. So sind im Chinese Journal of Medicinal Chemistry 5(4), 266-270 (1995), 3 Kristallmodifikationen beschrieben, die als Modifikationen A, B und C bezeichnet werden. Modifikation A wird bei der Umkristallisation von Doxazosin-Mesylat aus Ethanol gewonnen. Die Modifikationen B und C entstehen bei der Umkristallisation von Doxazosin-Mesylat aus Chloroform bzw. Wasser. Es sei darauf hingewiesen, daß im Chinese Journal of Medicinal Chemistry zwar lediglich von Doxazosin gesprochen wird. Nach den publizierten Daten muß es sich jedoch um das Doxazosin-Mesylat handeln. Die Modifikation A ist auch Gegenstand der EP-A 0 849 266, sie wird darin als Form III bezeichnet.

Eine weitere Modifikation ist in EP-A 0 848 001 beschrieben und charakterisiert worden, sie hat keinen besonderen Namen erhalten. In der EP-A 0 849 264 wird eine Form I und in der EP-A 0 849 265 eine Form II des Doxazosin-Mesylats beschrieben.

In der nicht vorveröffentlichten Patentanmeldung PCT/EP/98/08360 (angemeldet am 18.12.98) ist eine Form D des Doxazosin-Mesylats beschrieben worden, die als Zwischenprodukt bei der Herstellung der Form A des Doxazosin-Mesylats auftritt.

Es wurde nun gefunden, daß sich die Modifikation D des Doxazosin-Mesylats besonders gut als Arzneimittel gegen Bluthochdruck einsetzen läßt.

Gegenstand der Erfindung sind Arzneimittel, enthaltend die Modifikation D des Doxazosin-Mesylats sowie die Verwendung der Modifikation D des Doxazosin-Mesylats zur Herstellung von Arzneimitteln gegen Bluthochdruck.

Die Modifikation D des Doxazosin-Mesylats wird hergestellt, indem man Doxazosin mit Methansulfonsäure in Methanol löst, die so erhaltene Lösung gegebenenfalls von einer Trübung befreit und die dabei anfallende, klare Lösung so lange rührt, bis kein Niederschlag mehr ausfällt, den Niederschlag abtrennt und mit Methanol wäscht und trocknet.

Zur Umsetzung von Doxazosin mit Methansulfonsäure werden die beiden Substanzen im Molverhältnis von etwa 1:1 eingesetzt. Vorzugsweise wird ein geringer molarer Überschuß an der Sulfonsäure verwendet (bis etwa 10 %).

Falls die Zeit zwischen dem Erhalt einer Lösung durch Zugabe von Methansulfonsäure zum Doxazosin und dem Erscheinen eines Niederschlags nicht für eine Filtration ausreicht - beispielsweise wenn die Umsetzung im technischen Maßstab durchgeführt werden soll -, kann man die Zeit für die Filtration dadurch verlängern, daß man dem für die Umsetzung verwendeten Methanol ein aprotisches, polares organisches Lösungsmittel zusetzt.

Als aprotische, polare organische Lösungsmittel eignen sich in diesem Fall beispielsweise N,N-Dimethylformamid und insbesondere N-Methyl-2-pyrrolidon. Das Verhältnis von Doxazosin zu Methanol (Gewicht/Volumen) bzw. das Verhältnis von Doxazosin zu Methanol zu dem aprotischen, polaren organischen Lösungsmittel (Gewicht/Volumen/Volumen) beträgt etwa 1 : (5 bis 15), vorzugsweise etwa 1 : (8 bis 12) bzw. etwa 1 : (5 bis 15) : (1,5 bis 4), vorzugsweise etwa 1 : (8 bis 12) : (2 bis 3).

Soll bei dem Verfahren die nach der Zugabe der Methansulfonsäure erhaltene Lösung durch Filtrieren von gegebenenfalls vorhandenen Fremdpartikeln befreit werden, so wird bevorzugt mit dem Lösungsmittelgemisch aprotisches, polares organisches Lösungsmittel/Methanol gearbeitet. Grund ist, daß in diesem Falle, wie schon erwähnt, die Zeitspanne zwischen Erhalt der Lösung und Bildung erster Kristalle größer ist als bei alleiniger Verwendung von Methanol als Lösungsmittel. Wird bei dem Verfahren eine Filtration der nach der Zugabe der Methansulfonsäure erhaltenen Lösung gewünscht, so besteht eine besonders bevorzugte Vorgehensweise darin, daß in dem Lösungsmittelgemisch aus aprotischem, polarem organischem Lösungsmittel gearbeitet wird und darüber hinaus ein Teil des Methanols erst nach der Filtration zugegeben wird.

Die Modifikation D des Doxazosin-Mesylats ist insbesondere charakterisiert durch Hauptlinien im Debye-Scherrer Röntgen-Diffraktogramm bei folgenden, in Winkelgrad angegebenen Werten von 2 Theta: 5,72 ± 0,2°; 11,10 ± 0,2°; 11,46 ± 0,2°; 14,14 ± 0,2°; 17,01 ± 0,2°; 17,78 ± 0,2°; 18,33 ± 0,2°; 20,73 ± 0,2°; 21,70 ± 0,2°; 23,12 ± 0,2°; 24,28 ± 0,2°; 26,58 ± 0,2°.

Die Modifikation D des Doxazosin-Mesylats besitzt den Vorteil, daß sie sich leichter zu Tabletten verarbeiten läßt. So zeigt diese Modifikation eine sehr gute Mischbarkeit mit anderen Substanzen bzw. ein ausgezeichnetes Mischverhalten. Das sehr gute Fließverhalten der neuen Modifikation erleichtert weiter die Herstellung von festen galenischen Applikationsformen.

### Beispiele

### Herstellung von Doxazosin-Mesylat der Kristallmodifikation D

### Verfahren a

In einem 1-l-Dreihalsrundkolben wurde ein Gemisch aus 63,2 g Doxazosin, 125 ml N-Methyl-2-pyrrolidon und 500 ml Methanol unter Rühren mit 14,1 g wasserfreier Methansulfonsäure versetzt. Dabei stieg die Innentemperatur auf 30°C an, und es entstand eine Lösung. Nach beendeter Zugabe der Methansulfonsäure wurde der Reaktionsansatz in einen zweiten 1-l-Dreihalsrundkolben filtriert. Der Filter wurde mit 85 ml Methanol nachgewaschen und die vereinigten Filtrate 5 h gerührt. Nach beendeter Rührzeit wurde der entstandene Niederschlag abgesaugt und 3x mit je 25 ml Methanol gewaschen. Man erhielt 125 g feuchtes Doxazosin-Mesylat (Modifikation D). Das entspricht 70,4 g Trockensubstanz und einer Ausbeute von 91,8 %.

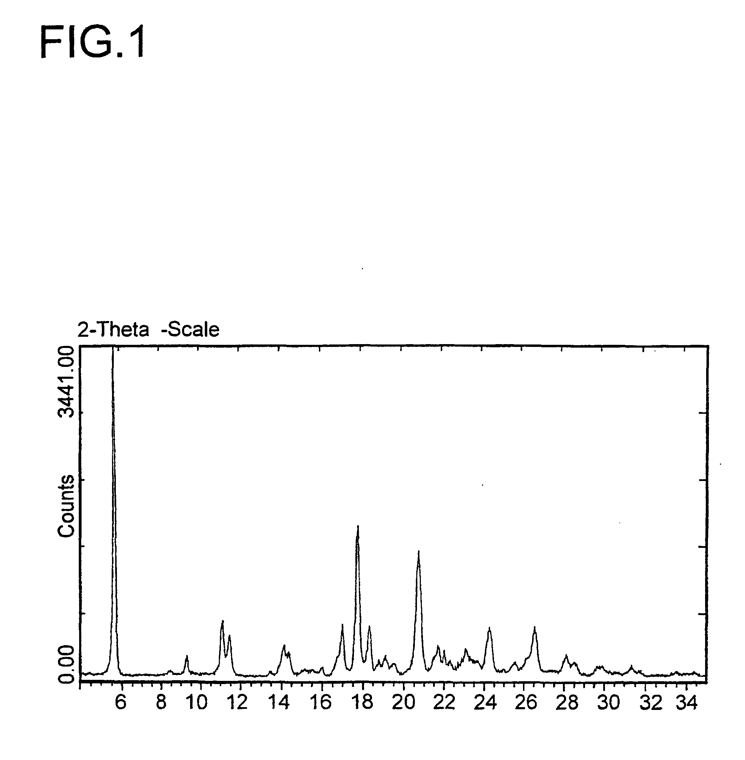

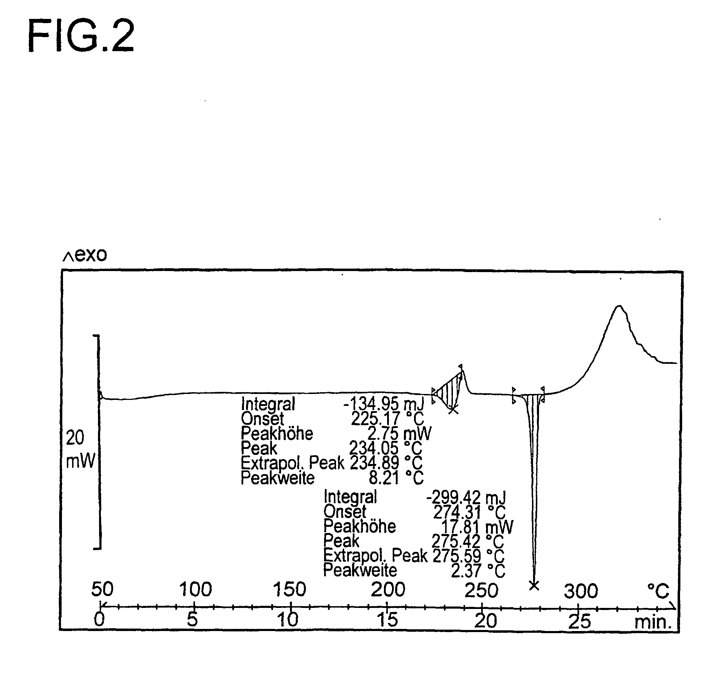

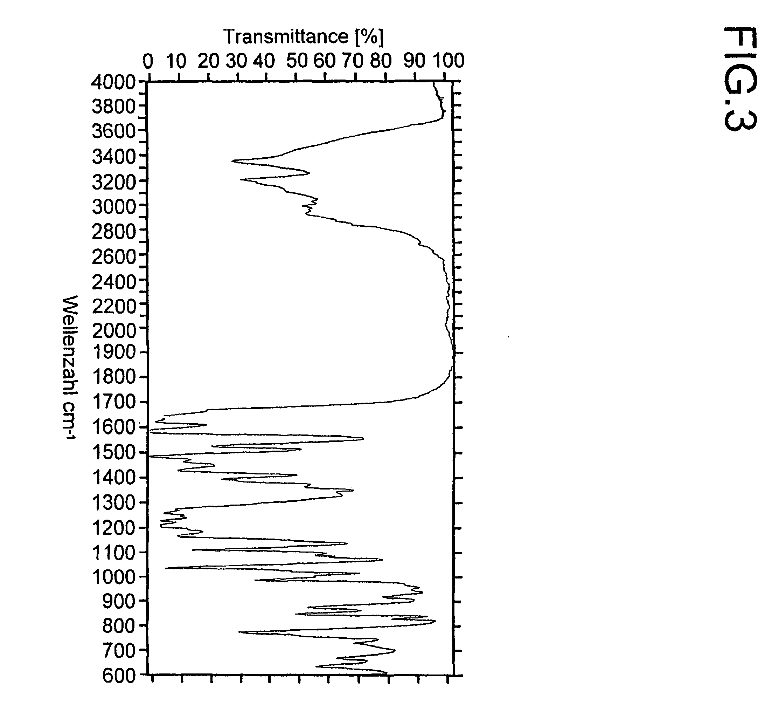

Die Modifikation D des Doxazosin-Mesylats wurde durch das Debye-Scherrer Röntgendiffraktogramm, durch das Differential Scanning Thermogramm und durch das IR-Spektrum charakterisiert (vgl. Abb. 1-3; für 2-Theta-Werte der Hauptlinien im Diffraktogramm siehe oben). Alle Daten wurden an im Vakuum getrocknetem Material erhoben.

### Verfahren b

In einem 500-ml-Dreihalsrundkolben wurde ein Gemisch aus 27,1 g Doxazosin, 54 mL N-Methyl-2-pyrrolidon und 250 ml Methanol unter Rühren mit 6,1 g wasserfreier Methansulfonsäure versetzt. Dabei stieg die Innentemperatur auf 30°C an, und es entstand eine Lösung. Nach beendeter Zugabe der Methansulfonsäure wurde der Reaktionsansatz in einen zweiten 500-ml-Dreihalsrundkolben filtriert und das Filtrat 5 h gerührt. Nach beendeter Rührzeit wurde der entstandene Niederschlag abgesaugt und 2x mit je 50 ml Methanol gewaschen. Man erhielt 45 g feuchtes Doxazosin-Mesylat (Modifikation D). Das entspricht 28,5 g Trockensubstanz und einer Ausbeute von 86,7 %.

### Verfahren c

In einem 2-l-Dreihalsrundkolben wurde ein Gemisch aus 79,0 g Doxazosin und 800 ml Methanol unter Rühren mit 17,7 g wasserfreier Methansulfonsäure versetzt. Dabei stieg die Innentemperatur auf 30°C an und es entstand eine Lösung. Nach beendeter Zugabe der Methansulfonsäure wurde noch 5 h nachgerührt. Dann wurde der entstandene Niederschlag abgesaugt und 3x mit je 50 ml Methanol gewaschen. Man erhielt 141,8 g feuchtes Doxazosin-Mesylat (Modifikation D). Das entspricht 89,2 g Trockensubstanz und einer Ausbeute von 93,1 %.

### Beispiele für galenische Applikationsformen

121 g Doxazosin-Mesylat (Form D), 5 g Aerosil® 200 (hochdisperse Kieselsäure) und 874 g sprühgetrocknete Lactose wurden miteinander vermischt und die so erhaltene Mischung über eine Laborstiftmühle gemahlen bzw. verrieben. Die Herstellung einer Verreibung war wichtig, damit die Tabletten eine gute Content Uniformity erhalten. Die so gewonnene Verreibung diente als Basis-Mischung für folgende Formulierungen:

### A. Tabletten, enthaltend 1 mg Doxazosin-Mesylat (Modifikation D)

10 g der Basis-Mischung wurden mit 89 g Ludipress® (Fertighilfsstoffpräparation der BASF Aktiengesellschaft) und 1 g Magnesiumstearat gemischt und auf einer Excenter-Presse in einem Tablettenstempel von 7 mm Durchmesser mit einer Preßkraft von 8 kN zu Tabletten mit einem Gewicht von 100 mg und einer Härte von 80N gepreßt. Der Wirkstoffgehalt pro Tablette betrug 1,21 mg Doxazosin-Mesylat entsprechend 1 mg Doxazosin-Base. Die so erhaltenen Tabletten zerfielen bei 20°C in Wasser innerhalb von 2 min. Die Wirkstoff-Freisetzung nach 20 min betrug 85 %.

### B. Tabletten, enthaltend 2 mg Doxazosin-Mesylat (Modifikation D)

Analog A. wurden Tabletten mit einem Gewicht von 200 mg hergestellt. Diese wurden in einem Stempelformat von 9 mm Durchmesser mit einer Preßkraft von 9 kN gepreßt und zeigten eine Härte von 60N. Der Gehalt der Tablette betrug 2,42 mg Doxazosin-Mesylat entsprechend 2 mg Doxazosin-Base. Die so erhaltenen Tabletten zerfielen bei 20°C in Wasser innerhalb von 3 min. Die Wirkstoff-Freisetzung nach 20 min betrug 81 %.

### C. Tabletten, enthaltend 4 mg Doxazosin-Mesylat (Modifikation D)

20 g der Basis-Mischung wurden mit 79 g Ludipress® (Fertighilfsstoffpräparation der BASF Aktiengesellschaft) und 1 g Magnesiumstearat gemischt und auf einer Excenter-Presse in einem Tablettenstempel von 9 mm Durchmesser mit einer Preßkraft von 8 kN zu Tabletten mit einem Gewicht von 200 mg und einer Härte von 50N gepreßt. Der Wirkstoffgehalt pro Tablette betrug 4,84 mg Doxazosin-Mesylat entsprechend 4 mg Doxazosin-Base. Die so erhaltenen Tabletten zerfielen bei 20°C in Wasser innerhalb von 4 min. Die Wirkstoff-Freisetzung nach 20 min betrug 80 %.

Die Bestimmung der Freisetzung in vitro erfolgte nach folgender Vorschrift:

Die Tabletten wurden in der Paddle-Apparatur entsprechend USP XXIII mit 6 einzelnen Freisetz-Gefäßen auf ihre Wirkstoff-Freisetzung geprüft. Die einzelnen Gefäße enthielten 900 ml einer 0,08 molaren HCl-Lösung. Die Rührgeschwindigkeit betrug 50 UpM. Die Temperatur lag bei 37°C. Die Anzahl der geprüften Tabletten betrug 6 Tabletten pro Dosierung. Nach 20 min wurden 100 ml Lösung entnommen, die durch einen Filter (0,5 µm) filtriert wurden. Diese Lösung wurde ohne weitere Verdünnung direkt zur Bestimmung des aus den Tabletten gelösten Anteils an Doxazosin-Mesylat eingesetzt. Die Bestimmung des Wirkstoffs erfolgte mit einem UV-Spektralphotometer bei der Wellenlänge 245 nm.

Die Soll-Extinktionen betragen bei quantitativer Lösung des Wirkstoffs: Dosis 1 mg: E_{Soll} 0,116, Dosis 2 mg: E_{Soll} 0,231 und Dosis 4 mg: E_{Soll} 0,462.

## Patentansprüche

1. Arzneimittel, enthaltend die Modifikation D des Doxazosin-Mesylats, charakterisiert durch Hauptlinien im Debye-Scherrer Röntgen-Diffraktogramm bei folgenden, in Winkelgrad angegebenen Werten von 2 Theta: 5,72 ± 0,2°; 11,10 ± 0,2°; 11,46 ± 0,2 °; 14,14 ± 0,2°; 17,01 ± 0,2°; 17,78 ± 0,2°; 18,33 ± 0,2°; 20,73 ± 0,2°; 21,70 ± 0,2°; 23,12 ± 0,2°; 24,28 ± 0,2°; 26,58 ± 0,2°.

2. Verwendung der Modifikation D des Doxazosin-Mesylats gemäß der Definition nach Anspruch 1 zur Herstellung von Arzneimitteln gegen Bluthochdruck.

## Claims

1. Medicament comprising the modification D of doxazosin mesylate **characterized by** lines in the Debye-Scherrer X-ray diffractogram at the following values of 2 theta (indicated in angular degrees): 5,72 ± 0,2°; 11,10 ± 0,2°; 11,46 ± 0,2 °; 14,14 ± 0,2°; 17,01 ± 0,2°; 17,78 ± 0,2°; 18,33 ± 0,2°; 20,73 ± 0,2°; 21,70 ± 0,2°; 23,12 ± 0,2°; 24,28 ± 0,2°; 26,58 ± 0,2°.

2. Use of modification D of doxazosin mesylate according to definition of claim 1 for the preparation of medicaments for the treatment of high blood pressure.

## Revendications

1. Médicament, contenant la modification D du mésylate de doxazosine, **caractérisé par** les lignes principales dans le diffractogramme à rayons X de Debye - Scherrer pour les valeurs de 2 théta suivantes indiquées en degrés d'angle,
: 5,72 ± 0,2° ; 11,10 ± 0,2° ; 11,46 ± 0,2° ; 14,14 ± 0,2° ; 17,01 ± 0,2° ; 17,78 ± 0,2° ; 18,33 ± 0,2° ; 20,73 ± 0,2° ; 21,70 ± 0,2° ; 23,12 ± 0,2° ; 24,28 ± 0,2 ; 26,58 ± 0,2°.

2. Utilisation de la modification D du mésylate de doxazosine selon la définition de la revendication 1 pour la préparation de médicaments contre l'hypertension artérielle.
